(19) Europäisches Patentamt
European Patent Office
Office européen des brevets

(11) Veröffentlichungsnummer: **0 085 884**
**A1**

(12) # EUROPÄISCHE PATENTANMELDUNG

(21) Anmeldenummer: **83100615.0**

(22) Anmeldetag: **25.01.83**

(51) Int. Cl.³: **C 07 D 307/62**
//C08F218/00

(30) Priorität: **06.02.82 DE 3204127**

(43) Veröffentlichungstag der Anmeldung:
**17.08.83 Patentblatt 83/33**

(84) Benannte Vertragsstaaten:
**DE FR GB**

(71) Anmelder: **BAYER AG**
**Zentralbereich Patente, Marken und Lizenzen**
**D-5090 Leverkusen 1, Bayerwerk(DE)**

(72) Erfinder: **Muisers, Hans-Ferdinand, Dr.**
**Hahnenweg 5**
**D-5000 Koeln 80(DE)**

(72) Erfinder: **Arlt, Dieter, Prof. Dr.**
**Rybniker Strasse 2**
**D-5000 Koeln 80(DE)**

(72) Erfinder: **Jautelat, Manfred, Dr.**
**Muellersbaum 28**
**D-5093 Burscheid(DE)**

(72) Erfinder: **Alberts, Heinrich, Dr.**
**Schulstrasse 1A**
**D-5068 Odenthal(DE)**

(72) Erfinder: **Mietzsch, Fritz, Dr.**
**Hauptstrasse 364**
**D-5060 Bergisch-Gladbach 2(DE)**

(54) **Acylanhydrozitronensäurevinylester und ihre Herstellung.**

(57) Acylanhydrozitronensäurevinylester der Formel

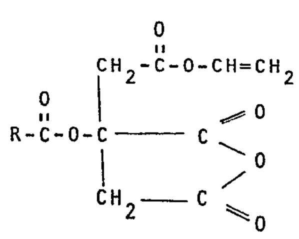

in der
R Wasserstoff oder Niederalkyl bedeutet,
ihre Herstellung und ihre Anwendung in der Polymerchemie.

**EP 0 085 884 A1**

0085884

BAYER AKTIENGESELLSCHAFT 5090 Leverkusen, Bayerwerk
Zentralbereich
Patente, Marken und Lizenzen
Mn/bc/c

# Acylanhydrozitronensäurevinylester und ihre Herstellung

Die vorliegende Erfindung betrifft neue Acylanhydrozitronensäurevinylester und ein Verfahren zu ihrer Herstellung.

Endprodukte, im besonderen in der Polymerchemie, basieren in der Regel auf Ausgangsstoffen, die aus dem Erdöl stammen. Bei der allgemeinen Verknappung der Erdölprodukte besteht bei der Entwicklung neuer Produkte ein Bedürfnis von Ausgangsstoffen auszugehen, die unabhängig vom Erdöl sind und sich immer wieder neu bilden.

Gegenstand der vorliegenden Erfindung sind neue Acylanhydrozitronensäurevinylester, die sich von der Zitronensäure ableiten. Zitronensäure wird durch Fermentation von Kohlenhydraten (z.B. Maisstärke oder Melasse) in großen Mengen hergestellt, und steht wegen der Herstellung aus natürlichen Abfallprodukten günstig zur Verfügung.

Die neuen Acylanhydrozitronensäurevinylester entsprechen der Formel (I)

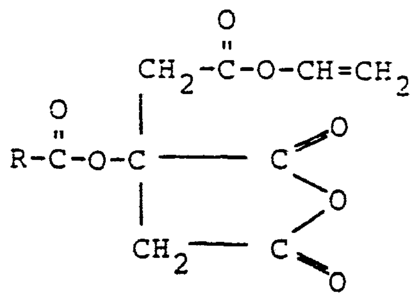

(I)

in der

R Wasserstoff oder Niederalkyl bedeutet.

Niederalkyl bedeutet erfindungsgemäß ein geradkettiger oder verzweigter Kohlenwasserstoffrest mit 1 bis etwa 6 Kohlenstoffatomen. Beispielsweise seien genannt: Methyl, Ethyl, Propyl, Isopropyl, Butyl, Isobutyl, Pentyl, Isopentyl, Hexyl und Isohexyl.

Bevorzugter Acylanhydrozitronensäurevinylester ist die Verbindung der Formel (II)

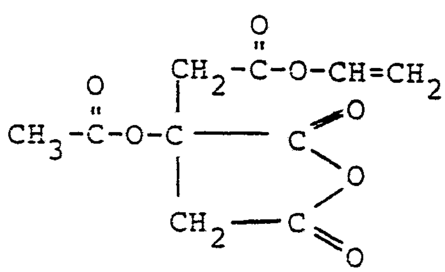

(II)

Acylanhydrozitronensäurevinylester können hergestellt werden, indem man Zitronensäure zunächst mit einem Carbonsäureanhydrid oder einem Carbonsäurechlorid umsetzt und anschließend in Gegenwart eines Katalysators mit Vinylacetat oder durch Einleiten von Acetylen umestert.

Das erfindungsgemäße Verfahren wird vorzugsweise als "Eintopfverfahren" durchgeführt.

Das erfindungsgemäße Verfahren kann beispielsweise durch die folgende Reaktionsgleichung erläutert werden:

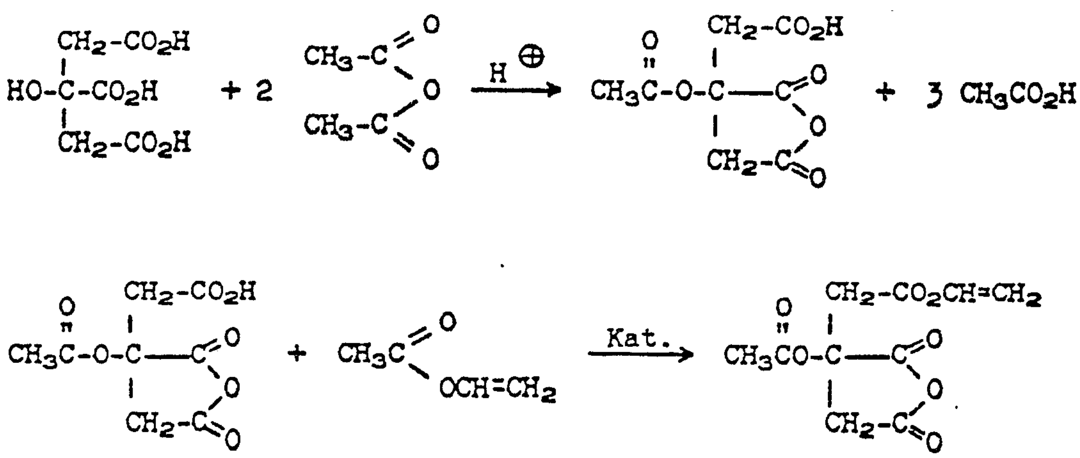


Carbonsäureanhydride für das erfindungsgemäße Verfahren sind die Anhydride niederer organischer Carbonsäuren. Sie können durch die Formel

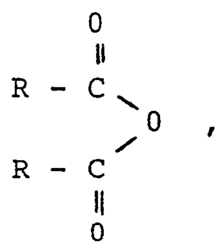

in der
R die obengenannte Bedeutung hat,
beschrieben werden.

Carbonsäurechloride für das erfindungsgemäße Verfahren sind die Säurechloride niederer organischer Carbonsäuren. Sie können durch die Formel

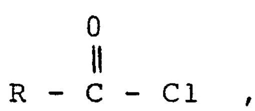

in der
R die obengenannte Bedeutung hat,
beschrieben werden.

Für das erfindungsgemäße Verfahren wird Acetanhydrid bevorzugt.

Das erfindungsgemäße Verfahren wird im allgemeinen im Temperaturbereich von 40 bis 110°C, vorzugsweise von 50 bis 70°C, durchgeführt. Im allgemeinen arbeitet man bei Normaldruck (etwa 1 bar) oder bei erhöhtem Druck (bis zu etwa 3 bar). Vorzugsweise wird das erfindungsgemäße Verfahren bei Normaldruck durchgeführt.

Die Durchführung der ersten Reaktionsstufe erfolgt erfindungsgemäß in der Weise, daß wasserfreie Zitronensäure mit der stöchiometrischen Menge oder einer überschüssigen Menge (bis zu 3 Mol/Mol Zitronensäure) des Carbonsäureanhydrids oder des Carbonsäurechlorids, vorzugsweise der stöchiometrischen Menge des Carbonsäureanhydrids, im erfindungsgemäßen Temperaturbereich umgesetzt wird. Es ist vorteilhaft, geringe Mengen eines sauren Katalysators zuzufügen. Saure Katalysatoren sind beispielsweise konzentrierte Schwefelsäure, Phosphorsäure oder Bortrifluoridetherat. Die Menge des Katalysators beträgt erfindungsgemäß im allgemeinen 0,01 bis 0,1 Gew.-%, bezogen auf die Zitronensäure. Vozugsweise setzt man 0,01 bis 0,05 Gew.-% des Katalysators, bezogen auf die Zitronensäure, ein. Nach Beendigung der Umsetzung wird die entstandene Essigsäure unter Normaldruck (etwa 1 bar) oder vermindertem Druck (bis zu etwa 1 mbar), bevorzugt unter vermindertem Druck, abdestilliert.

Der Rückstand des ersten Reaktionsschrittes wird im zweiten Reaktionsschritt erfindungsgemäß mit Vinylacetat in Gegenwart eines Katalysators umgesetzt. Als geeignete Katalysatoren seien Palladium- oder Quecksilbersalze, bevorzugt Quecksilbersalze, genannt. Als Salze werden im allgemeinen die Halogenide (Fluoride, Chloride, Bromide und Jodide, bevorzugt die Chloride) und die Acetate eingesetzt. Beispielsweise seien genannt: Palladium-II- und Quecksilber-II-chlorid, Palladium-II- und Quecksilber-II-acetat. Vorzugsweise wird Quecksilber-II-acetat eingesetzt.

Die Menge des Katalysators im zweiten Reaktionsschritt beträgt 0,1 bis 10 Gew.-%, bezogen auf die Menge der eingesetzten Zitronensäure. Bevorzugt wird eine Menge von 0,1 bis 1 Gew.-% eingesetzt.

Vorteilhafterweise führt man das erfindungsgemäße Verfahren mit einem Überschuß von Vinylacetat durch. Im allgemeinen verwendet man 2 bis 10 Mol Vinylacetat, bevorzugt 3 bis 5 Mol Vinylacetat, pro Mol eingesetzter Zitronensäure.

Nach Beendigung der zweiten Reaktionsstufe wird der Katalysator als schwerlösliches Sulfid ausgefällt und kann so nahezu quantitativ abgetrennt werden. Die Fällung erfolgt durch Zugabe einer äquimolaren Menge einer geeigneten Schwefelverbindung. Geeignete Schwefelverbindungen sind solche, die sich in Vinylacetat und Essigsäure in nennenswertem Umfang lösen und mit dem Katalysator ein Sulfid bilden.

Geeignete Fällungsmittel für den Katalysator sind z.B. Schwefelwasserstoff, Metallsulfide, z.B. Natriumsulfid, organische Thioverbindungen, z.B. Thiocarbonsäureamide, Thioharnstoff und alkylierte Thioharnstoffe. Besonders bevorzugt wird Thioacetamid eingesetzt.

Die Bildung eines filterfähigen Quecksilbersulfidniederschlags erfolgt im allgemeinen langsam. Es empfiehlt sich daher, die Zugabe des Fällungsmittels bei der erfindungsgemäßen Reaktionstemperatur durchzuführen und bei dieser Temperatur noch 1 bis 5 Stunden, vorzugsweise 2 bis 3 Stunden, weiterzurühren.

Die Abtrennung des Quecksilbersulfidniederschlags erfolgt in der Weise, daß die warme Reaktionslösung über ein geeignetes Filtrierhilfsmittel abgesaugt wird. Als Filtrierhilfsmittel kommen Aktivkohle, Kieselgel, Kieselgur und Bleicherde, bevorzugt Bleicherde, in Frage.

Beim Abkühlen des Filtrats fällt das gewünschte Produkt zu einem großen Teil aus und kann in an sich bekannter Weise, z.B. durch Absaugen, abgetrennt werden.

In einer veränderten Ausführungsform des erfindungsgemäßen Verfahrens kann man nach Beendigung der ersten Reaktionsstufe die Reaktionslösung mit dem Katalysator versetzen und unter Druck (bevorzugt bis zu 3 bar) oder bei Normaldruck, bevorzugt bei Normaldruck, Acetylen einleiten. In diesem Fall beträgt die Menge an Katalysator bevorzugt 0,1 bis 10 Gew.-%, insbesondere bevorzugt 0,2 bis 2 Gew.-%, bezogen auf die Menge der eingesetzten Zitronensäure.

Bei der Einleitung von Acetylen in die zweite Reaktionsstufe führt man diese bevorzugt im Temperaturbereich von 0 bis 100°C, besonders bevorzugt bei Raumtemperatur (bei etwa 20°C), durch.

Acylanhydrozitronensäurevinylester lassen sich mit verschiedenen Monomeren copolymerisieren. Als Monomere seien beispielsweise genannt: Vinylverbindungen wie Styrol, Vinylchlorid oder die Carbonsäureester von Vinylalkohol, Derivate der (Meth-)acrylsäure, insbesondere deren Ester, Ethylen und alpha-Olefine.

Durch Copolymerisation der erfindungsgemäßen Acylanhydrozitronensäurevinylester mit den an sich bekannten Monomeren erhält man neue Polymere, die eine reaktive Carbonsäureanhydridgruppierung enthalten.

Polymere, die eine Carbonsäureanhydridgruppierung enthalten, wurden bisher durch Copolymerisation mit Maleinsäureanhydrid hergestellt (Literatur: Houben-Weyl "Methoden der Organischen Chemie" Band XIV/1, Georg Thieme-Verlag, Stuttgart). Maleinsäureanhydrid wird bei der Copolymerisation im Molverhältnis 1 : 1 und alternierend in das Polymere eingebaut. Für die meisten anwendungstechnischen Zwecke ist dies von Nachteil. Erwünscht sind niedrigere Einbauarten und eine statistische Verteilung von Anhydridgruppen, da sonst das Polymer versprödet und wasser- bzw. alkaliempfindlich wird.

Bei der Copolymerisation der erfindungsgemäßen Acylanhydrozitronensäurevinylester wird diese Komponente nicht alternierend eingebaut. Es ist so in überraschender Weise möglich, die Menge an Acylanhydrozitronensäurevinylester-Baustein in weiten Grenzen festzulegen.

Die neuen Polymere lassen sich aufgrund ihrer Reaktionsfähigkeit vielfältig einsetzen; so können sie zum Beispiel zur Verbesserung der Haftung in Klebern, als Vernetzer in Lacksystemen und Duromeren oder nach Ringöffnung mit Basen als Dispergierhilfsmittel bzw. als Viskositätserhöher in wäßrigen Systemen eingesetzt werden.

Beispiel 1

3630 g (15 Mol) wasserfreie Zitronensäure, 3060 g (30 Mol) Acetanhydrid und 0,5 ml konz. Schwefelsäure werden bei ca. 60°C etwa 6 Stunden gerührt. Die entstandene Essigsäure wird im Wasserstrahlvakuum nahezu vollständig abdestilliert. Der erstarrte Rückstand wird mit 3870 g (45 Mol) Vinylacetat und 24 g Quecksilber-II-acetat versetzt und bei ca. 60°C gerührt. Nach 12 Stunden fügt man 5,7 g Thioacetamid hinzu, rührt bei gleicher Temperatur noch etwa 2 Stunden weiter und saugt dann über Tonsil ab. Der Quecksilbergehalt des Filtrates beträgt ca. 1 ppm.

Aus dem Filtrat fallen nach einiger Zeit 1630 g (45 %) des gewünschten Produktes aus. Es kann in Toluol umkristallisiert werden (Fp.: 108°C).

Beispiel 2

1 Mol wasserfreie Zitronensäure und 2 Mol Acetanhydrid werden nach Zugabe eines Tropfens konz. Schwefelsäure bei 60°C etwa 6 Stunden gerührt. Die entstehende Essigsäure wird im Wasserstrahlvakuum nahezu vollständig abdestilliert. Der erstarrte Rückstand wird mit 4 Mol Vinylacetat und 3,5 g Palladium-II-chlorid versetzt und 8 Stunden unter Rückfluß gekocht. Nach Beendigung der Reaktion werden 15 g Aktivkohle zugefügt und die Reaktionsmischung heiß filtriert. Das Filtrat wird eingeengt. Der erstarrte Rückstand enthält laut Kernresonanzspektrum ca. 10 % Acetylanhydrozitronensäurevinylester.

Beispiel 3

1 Mol wasserfreie Zitronensäure und 2 Mol Acetanhydrid werden nach Zugabe eines Tropfens konz. Schwefelsäure 6 Stunden bei 60°C gerührt. Anschließend werden 2 g Quecksilber-(II)-acetat und 2 g Bortrifluorid-Etherat zugefügt und bei Raumtemperatur Acetylen eingeleitet. Die Reaktionstemperatur sollte 30°C nicht überschreiten. Die Reaktionslösung färbt sich dunkelbraun bis schwarz. Nachdem etwa 2 Mol Acetylen eingeleitet worden sind, saugt man den Niederschlag ab und wäscht ihn mit wenig kaltem Vinylacetat.

Man isoliert 72 g Acetylanhydrozitronensäurevinylester.

Beispiel 4

Ein 1 l-Hochdruckautoklav wird beschickt mit 88 g Acetylanhydrozitronensäurevinylester, 207 g Ethylacetat und 350 mg Azobisisobutansäurenitril. Nach Verdrängen der Luft mit Ethylen wird 12 Stunden auf 70°C erhitzt und dabei ein Ethylendruck von 300 bar dauernd aufrechterhalten. Man erhält eine Polymersuspension, die nach Filtration und Trocknen 57 g Polymer ergibt. Das Molverhältnis Acetylanhydrozitronensäurevinylester/Ethylen im Polymer beträgt laut Elementaranalyse 0,29 : 1.

Beispiel 5

Man verfährt wie in Beispiel 4, jedoch mit 14,5 g

Acetylanhydrozitronensäurevinylester und 300 g tert.-Butanol anstelle von Ethylacetat. Man erhält hier 40 g eines Polymers, das Acetylanhydrozitronensäurevinylester-Baustein im Molverhältnis 0,08 : 1 enthält.

Beispiel 6

Die Beispiele 4 und 5 werden wiederholt, jedoch mit Maleinsäureanhydrid statt Acetylanhydrozitronensäurevinylester. Ausbeute 97 bzw. 16 g Polymer; Molverhältnis Maleinsäureanhydrid/Ethylen 0,96 : 1 in beiden Fällen.

Patentansprüche

1) Acylanhydrozitronensäurevinylester der Formel

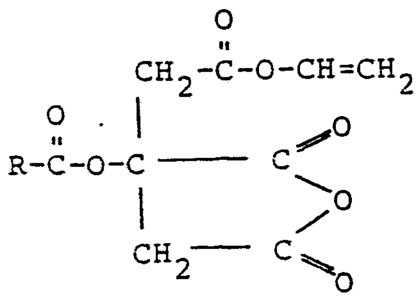

in der

R Wasserstoff oder Niederalkyl bedeutet.

2) Acylanhydrozitronensäurevinylester nach Anspruch 1 der Formel

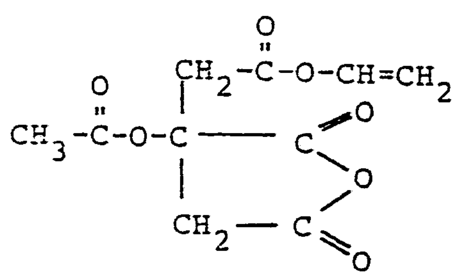

3) Verfahren zur Herstellung von Acylanhydrozitronensäurevinylester, dadurch gekennzeichnet, daß man Zitronensäure zunächst mit einem Carbonsäureanhydrid oder einem Carbonsäurechlorid umsetzt und anschließend unter Einwirkung eines Katalysators mit Vinylacetat oder durch Einleiten von Acetylen umestert.

4) Verfahren nach Anspruch 3, dadurch gekennzeichnet, daß man als Katalysator Quecksilber-II- oder Palladium-II-salze einsetzt.

5) Verfahren nach den Ansprüchen 3 bis 4, dadurch gekennzeichnet, daß man im Temperaturbereich von 40°C bis 110°C arbeitet.

6) Verfahren nach den Ansprüchen 3 bis 5, dadurch gekennzeichnet, daß man nach Beendigung der Umsetzung den Quecksilberkatalysator mit Hilfe einer Schwefelverbindung als Quecksilbersulfid ausfällt.

7) Verfahren nach den Ansprüchen 3 bis 6, dadurch gekennzeichnet, daß man die Schwefelverbindung mit Thioacetamid ausfällt.

8) Verfahren nach den Ansprüchen 3 bis 7, dadurch gekennzeichnet, daß man in eine Lösung von Acetylanhydrozitronensäure in Essigsäure in Gegenwart von Bortrifluorid-Etherat oder Bortrifluorid-Essigsäureaddukt und Quecksilber-II-salzen Acetylen einleitet.

Europäisches Patentamt

# EUROPÄISCHER RECHERCHENBERICHT

0085884
Nummer der Anmeldung

EP 83 10 0615

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | GB-A- 961 960 (STANDARD OIL) * Insgesamt * | 1-8 | C 07 D 307/62 // C 08 F 218/00 |
| Y | US-A-2 472 434 (A. PECHUKAS) * Insgesamt * | 1-8 | |
| Y | GB-A- 863 792 (MILES LAB.) * Insgesamt * | 1-8 | |
| Y | GB-A- 674 710 (EMULSOL CORP.) * Insgesamt * | 1-8 | |
| | | | RECHERCHIERTE SACHGEBIETE (Int Cl 3) |
| | | | C 07 D 307/00 |

Der vorliegende Recherchenbericht wurde für alle Patentansprüche erstellt.

| Recherchenort | Abschlußdatum der Recherche | Prüfer |
|---|---|---|
| DEN HAAG | 25-04-1983 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82